# EUROPEAN PATENT SPECIFICATION

(11) **EP 3 726 217 B1**
(45) Date of publication and mention of the grant of the patent: **28.06.2023**
(21) Application number: 20169514.5
(22) Date of filing: 15.04.2020
(51) Int. Cl.: G01N 33/543, G01N 21/64

(54) **EVANESCENCE BIOSENSOR OPTIMISATION**
OPTIMIERUNG EINES EVANESZENZ-BIOSENSORS
OPTIMISATION DE BIOCAPTEUR D'ÉVANESCENCE

(30) Priority: 18.04.2019 CH 5412019
(43) Date of publication of application: 21.10.2020
(73) Proprietor: Elionova AG, 1700 Fribourg (CH)
(72) Inventor: SCHAWALLER, Manfred, 1700 Fribourg (CH); QUAPIL, Gerald, 73277 Owen/Teck (DE)
(74) Representative: Müller-Boré & Partner Patentanwälte PartG mbB

(56) References cited:
- EP-A1- 3 460 456
- WO-A1-2008/099339
- JP-A- 2019 039 993
- US-A1- 2007 262 265
- US-A1- 2016 047 746
- M. SCHAWALLER ET AL: "Evanescent wave-based technology for the rapid detection and sensitive quantification of biological analytes", J. ALLERGY CLIN. IMMUNOL., vol. 141, no. 2, 23 October 2017 (2017-10-23), pages 817-820, XP055720305, DOI: 10.1016/j.jaci.2017.08.037
- YVES MERIEUX ET AL: "Evaluation of Diagnostic Tests by Evanescence Biosensor Technology for Rapid Phenotyping of the Human Platelet Alloantigens 1a and 5b", TRANSFUSION MEDICINE AND HEMOTHERAPY, vol. 46, no. 6, 30 October 2018 (2018-10-30), pages 440-445, XP055720309, CH ISSN: 1660-3796, DOI: 10.1159/000493556
- DUNCAN HILL ET AL: "Novel disposable biochip platform employing supercritical angle fluorescence for enhanced fluorescence collection", BIOMEDICAL MICRODEVICES, KLUWER ACADEMIC PUBLISHERS, BO, vol. 13, no. 4, 11 May 2011 (2011-05-11), pages 759-767, XP019921915, ISSN: 1572-8781, DOI: 10.1007/S10544-011-9546-2
- HANNU S VÃ LIMÃ KI ET AL: "Applying Total Internal Reflection Excitation and Super Critical Angle Fluorescence Detection to a Morphine Assay", JOURNAL OF FLUORESCENCE, KLUWER ACADEMIC PUBLISHERS-PLENUM PUBLISHERS, NE, vol. 20, no. 5, 13 April 2010 (2010-04-13) , pages 1003-1008, XP019822909, ISSN: 1573-4994

## Description

### Technical domain

The present invention concerns a test method and a test device, adapted for diagnoses. In particular it relates to immunoassay having an improved sensitivity compared to the known immunoassays.

### Related art

Examining human blood for specific markers is a commonly used method supporting medical diagnoses. A blood sample is taken from the patient and then assayed in a laboratory for the presence or absence of a diagnostic marker or a quantification of a marker. To distinguish from - in vivo - diagnostic procedures done on the patient him/herself laboratory tests are known as in vitro diagnostics commonly abbreviated as IVD. A commonly used technology of IVD tests is ELISA (Enzyme Linked immune Sorbent Assay) used to determine numerous markers in biological fluids and as such supporting a medical diagnosis. The ELISA and related technologies are in widespread use. All ELISA use a solid well of about 200 microliter volume to examine a patient sample. These well are made from organic polymer such as polystyrene by an injection molding process.

Typical assay formats used for immunological ELISA-assays are sandwich assays, competitive assays or antibody assays. A sandwich assay uses two ligands, usually a pair of antibodies both specific for the protein to be measured, examples are tests for Beta Human Chrionic Gonadotropin, beta HCG, other hormones such a Follicle Stimulating Hormone (FSH), Luteinizing Hormone (LH), the infection markers C-Reactive Protein CRP or Procalcitonin PCT, marker proteins for heart attack such as Troponin I und Troponin T or Myoglobin.

Further well know analytes are low molecular weight compounds like steroids, drugs, drug of abuse or vitamins. The assays are then designed as competitive assays.

Antibody assays are for example the detection of anti viral antibodies, directed against proteins of viruses such as HIV, HBV of HCV, or the anti bacterial or anti parasite antibodies. Furthermore antibody tests are used for the measurement of auto immune antibodies or allergy related IgE.

The principles of different ELISA formats are comparable. One Ligand is bound to the inside of an ELISA well. A second ligand is in solution and can react with the bound ligand or with an analyte that has bound to the immobilised ligand. For an ELISA the ligand in solution is covalently coupled to an enzyme such as horseradish peroxidise or alkaline phosphatase

Diffusion makes the labelled ligand in solution to move to the well surfaces and reacts with the surface bound ligand. A typical assay is done that after a certain time of reaction, 30-120min, the analyte solution with excess labelled ligand is removed and the well are washed with a buffer solution.

The bound ligand-enzyme conjugate bound to the well is measure by reacting it with a suitable chromogenic substrate giving a coloured product suitable for quantitation.

Advantages of ELISA are a high sensitivity and specificity. The analytical detection limits are given by the ligands and their affinities. An important component for the quality are the compositions of the buffers used and analytes in the range from 10exp-6 mol/L and 10exp-12 mol/L can be measured, in exceptional case detection limits of 10exp-13 mol/L have been reached. Taken all advantages of ELISA so is the need for numerous working steps a disadvantage. Several pipetting and washing steps to remove excess unbound ligands are required and the time-to-result is typically in the range of hours before a result can be read. During this time a treating doctor has to wait for a IVD result that will support or dismiss his clinical diagnosis and therefore a directed evidence base therapy is delayed

An improved method for clinical analytes is the use of biosensors. One popular method is the use of evanescence field excitation of bound analytes and detection of bound molecules in real time as described in EP1079226, EP1204856, EP1371967. Applications in for assays are described in EP2 639 584 and also for example by Schawaller et al. J Allergy Clin Immunol. 2018.

The method relates to a heterogenous immuno assay format similar to ELISA and relies on the Interaction of a surface bound ligand reacting in a non-covalent manner with at least one ligand from the solution in contact with the surface. The reaction takes place in a well produced from a solid optical transparent material such as glass or preferably made from an organic polymer such as polystyrene by injection molding. Whereas the ligand in the solution for an ELISA is labelled with an enzyme which in the development phase reveals in a quantitative manner bound enzyme and hence analyte in ELISA, the evanescence biosensor uses fluorescence detection as the measuring principle.

The ligand in solution is fluorescently labelled and when bound to the surface of the detector surface the marker is excited with a suitable light source, preferably of 635 nm wavelength, and the emitted fluorescence is continuously monitored using a dedicated instrument. The method allows the real-time continuous monitoring of a biochemical binding reaction. Several advantages come with this such as a specific excitation of bound fluorescently labelled ligand and hence analytes in the presence of excess non-bound ligand in the solution. A sensitive and quantitative result is measured typically after 10 to 20 min or in more favourable setting even less than 10 minutes time whereas an ELISA result is available only after hours.

Another advantage is that after adding the patient sample and the fluorescent second ligand no further liquid manipulation steps as in ELISA are required. Notably, washing steps and addition of enzyme substrates are not required. The method uses aqueous solutions such as PBS, neat or with additional reagents as for example detergents, surfactants, proteins or reaction enhancers. Samples to be measured can be urine saliva, serum plasma or detergent solubilised blood.

The major advantage of the technology lies in the fact that after the addition of all reagent and for the readout of the binding reaction there are all reagent in the well and the signal is measured in the presence of excess detection reagent present in the measurement well. This is unlike ELSIA where the added excess detector reagent has to be removed from the well before a detector substrate can be used to visualize the specifically bound detector agent. This is usually done by removing the excess detector reagent followed by several washing steps to remove essential all non bound detector reagent present in excess form the well. The amount of detector ligand when compared over the signal generating bound detector ligand is larger than 1. In fact, it is more than 10 folds present and can in fact be several orders of magnitude higher than the actual amount of analyte bound signal detector.

The document EP3460456 describes an optical device with a photodetector comprising a stack of interference filters.

The document WO2008099339 discloses a biosensor adapted for fluorescent radiations.

The document JP2019039993 describes a biosensor wherein the emitted light is filtered according to its incident angle.

The document from M Schawaller et all. "evanescent wave-based technology for the rapid detection and sensitive quantification of biological analytes" J. Allergy Clin. Immunol. Vol.141, n°2, pages 817-820 provides general information on biosensing methods.

Measuring a small specific signal in the background of a large amount of excess detector in a technical challenging task and careful optimisation of reducing the non-specific signal of excess reagent remains to be solved.

### Short disclosure of the invention

An aim of the present invention is the provision of a device that overcomes the shortcomings and limitations of the state of the art.

Another aim of the invention is the provision of a method allowing to detect a bound ligand in the presence of non-bound ligand.

The present invention further aims at providing a fast and reliable method, allowing to reduce the number of steps compared to the known methods.

In particular, the present method allows an optimal reduction of straylight.

According to one aspect, the present method and device allow to reduce the intensity of the excitating light that is measured in the detection unit. For example in the presented design the optical path of exciting lights is not directed directly towards the detector unit. Furthermore an aperture may limit the amount of excitation light that radiates towards the detector. Yet another suitable way to reduce the intensity of excitating light entering the detector surface is to make use of the different wavelength of excitation light EXC on the basis that the emitted fluorescent light has a different wavelength. The excitation light of 635 nm wavelength generates in when being absorbed by the fluorophore an emitted light having a different wavelength compared to the excitation light. In particular, it may have a longer wavelength. This can be favourably used to filter out the exciting photons based on the energy as a discriminator and hence as the wavelength a discriminator. This is typically achieved by using a wavelength specific filter and bandpass filters. Such bandpass filters or interference filters may be commercially readily available.

These filters are put into the emitting light beam in such a way that they are perpendicular arranged at β =90 degree angle with respect to the emitted light directed toward the detection unit. Alternatively, one or several of the optical filters are tilted at an angle different from 90°C with respect to the optical path.

The presently claimed device is adapted to detect the presence of fluorescent complexes of molecules and ligands at an emitted wavelength, said device comprises a light emitting beam adapted to irradiate the fluorescent complexes at said emitted wavelength, and a detector adapted to detect the fluorescence of the complexes. The claimed device further comprises at least one optical filter allowing to specifically absorb the wavelengths of the emitted light. The term "absorb" should be understood as partly absorb or reduce the intensity of the emitted light.

Preferably, the present device allows to detect the fluorescent complexes of the test molecules and bounded ligand in the presence of non-bounded fluorescent ligand. The non-bounded fluorescent ligands include the ligand remaining in the test solution or uncovalently bound on the support, at sites different from the tested molecule.

The at least one optical filter 101, 201 of the present device may be arranged so that the plane of its surface is orthogonal to the light pathway. In a preferred arrangement, the present device comprises a first optical filter, adapted to specifically absorb or attenuate the emitted light wherein said first optical filter is arranged at an angle orthogonal to the optical pathway. Alternatively, the first optical filter may be arranged so that its plan surface has an angle with regard to the light pathway larger than 10 degrees and different from 90 degrees.

The present device further comprises two or more individual optical filtering units absorbing the emitted light at the wavelength of said emitted light. These individual filtering unit may be optical filters identical to the first optical filter or different. They are preferably specific to the wavelength of the emitted light.

In a preferred embodiment, the individual filtering units, arranged in addition to the first optical filter, are oriented so that the plane of their surface is tilted with regard to the optical pathway at an angle larger than 10 degrees, preferably larger than 20 degrees, and different from 90. The individual filtering units are preferably placed after the first optical filter in the pathway of the emitted light. The individual filtering units have both a angular orientation different from the first optical filter. The orientation of each of these individual filtering units preferably differs from each other.

In particular, the tilting angles of the individual filtering unit are rotated against each other by more than 30 degrees with respect to the optical axis. More particularly, the tilting angles of the individual filtering unit are rotated against each other by more than 15 degrees and less than 180 degrees, or by more than 30 and less than 120 degrees. The tilting angles of the individual filtering units may be for example rotated against each other by an angle of 90 degrees.

The present disclosure also encompasses a method of detection of a complex of a tested molecule and a specific ligand bounded to said tested molecule. In the present method, the complex comprising the tested molecule and the ligand is immobilized on a surface. The present method thus comprises a step of immobilizing the complex of the tested molecule and its ligand. This immobilizing step may for example comprise the step of presenting a surface which contains at least one reaction partner L1 bound to the surface, followed by contacting said surface with a solution containing the molecule to be tested and a fluorescently labelled ligand L2. The fluorescently labelled ligand binds on the ligand L2 immobilized on the surface, either alone or in combination with the molecule to be tested to form a complex.

The present method includes an irradiation step, wherein the surface comprising the immobilized complex of tested molecule and its fluorescent ligand is irradiated at a predetermined wavelength. In this irradiation step, the intensity of the emitted light is attenuated by means of one or more optical filters, arranged as above-described. This irradiation step may occur without washing steps, meaning that the mixture comprising free fluorescent ligands, fluorescent ligand bounded alone on the surface and complexes of immobilized ligand and teste molecules is subject to the irradiation.

The resulting fluorescence is then detected in a detection step, using an adequate optical detector. The optical detector may be specific from the wavelength of the fluorescent complex of tested molecule and its ligand. The wavelength of the fluorescent complex of tested molecule and its ligand may be identical of the wavelength of the emitted light or different from the wavelength of the emitted light. The wavelength of the fluorescent complex of tested molecule and its ligand is preferably different from the wavelength of the emitted light which is attenuated by the device above-described.

### Short description of the drawings

Exemplar embodiments of the invention are disclosed in the description and illustrated by the following drawings:
- Figure 1 : Schematic view of the device according to the present invention;
- Figures 2a, 2b, 2c : Schematic view of the device according to the present invention according to an embodiment;
- Figures 3a, 3b, 3c : Schematic view of the device according to the present invention according to another embodiment;
- Figure 4: Schematic view of the device according to the present invention according to another embodiment.

### Examples of embodiments of the present invention

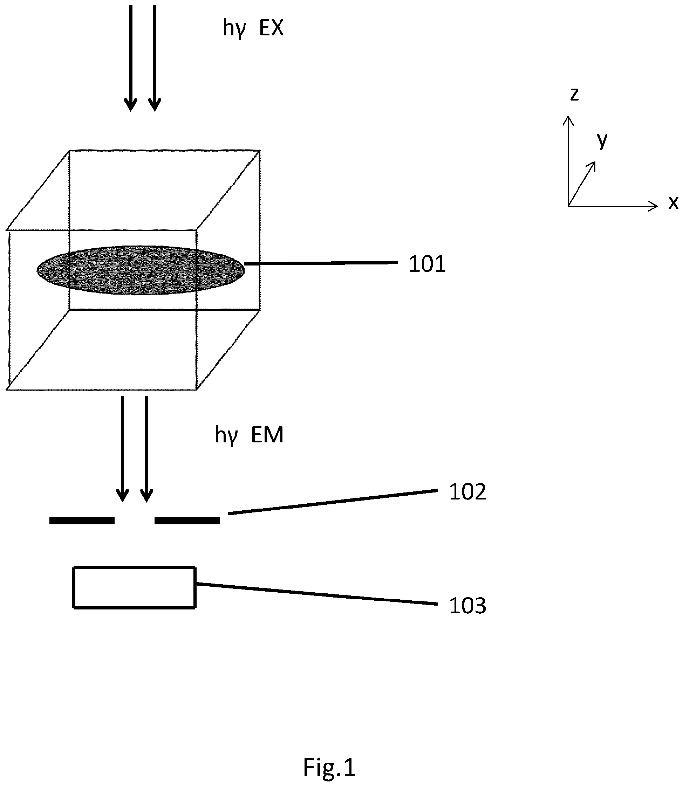

Fig. 1 shows the basic optical unit used. Emitted light from the biosensor device hγ **EX** arrives at the interference filter **101.** This filters out a majority of exciting photons based on the wavelength and the light leaving the interference filter at the bottom is enriched in hγ **EM** light which constitutes the useful signal for the fluorescing analyte. The useful signal should be understood as the signal specific from the bounded ligand. Passing through an optional aperture **102** the light is then measured using the light detector **103.** This arrangement is a functioning arrangement but further improvements are possible.

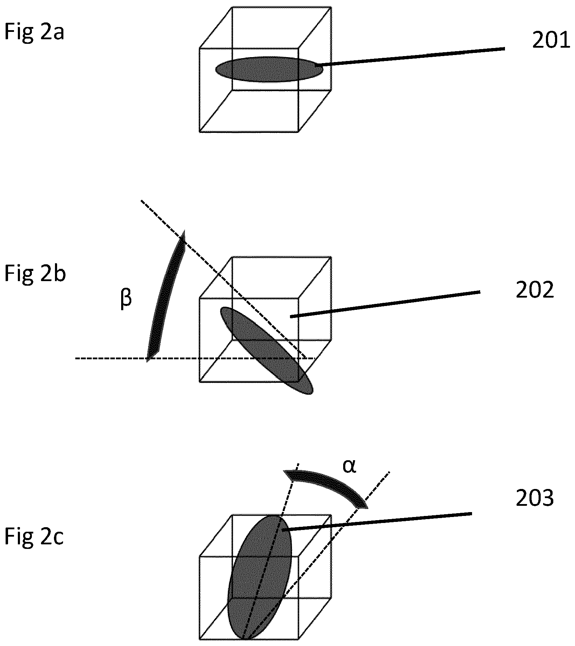

Figure 2 shows possible alternative arrangements of interference filters. Fig 2a shows a planar filter, fig 2b shows a tilted filter **202** with respect to the light beam from figure 1 and yet figure 2c shows a second tilted filter **203.** The second tilted filter differs from the filter **202** rotated that is in the xy axis by 90 degrees so that the tilting axes form an angle of 90 degrees when projected onto the xy axis. The angles β and the angle α by which the filters are tilted against the xy surface can be chosen freely. Preferably α = β > 10 degree and α = β < 80 degree, most preferably α = β is between 10 and 80 degrees, more preferably between 20 and 60 degrees and even more preferably between 30 and 45 degrees.

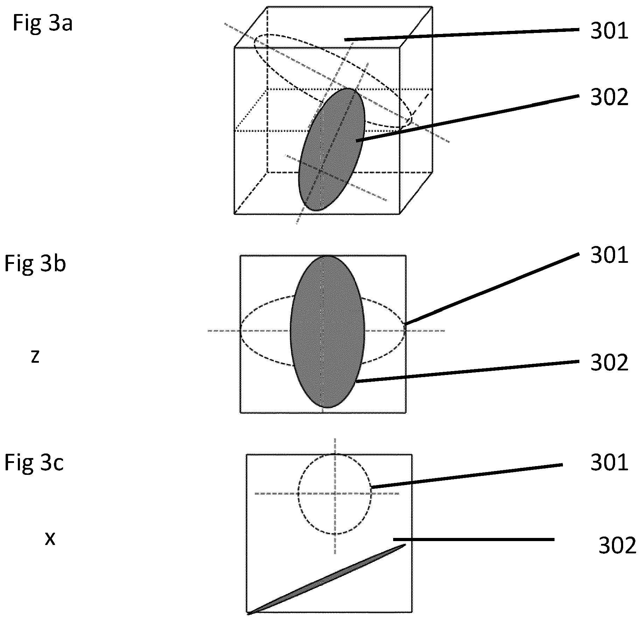

Fig 3 shows a possible combination of 2 interference filters as shown in figure 2. The filters depicted in fig 2b designated **202** and filter **203** fig. 2c are combined to a one interference filter unit consisting of 2 filters **301** and **302.** Fig 3a shows a three dimensional view of the filter arrangement. Figure 3b shows the same filter arrangement from the top view along the z axis looking down to the xy surface and fig 3c show the side of the same unit form the right side in direction x axis onto the yz surface.

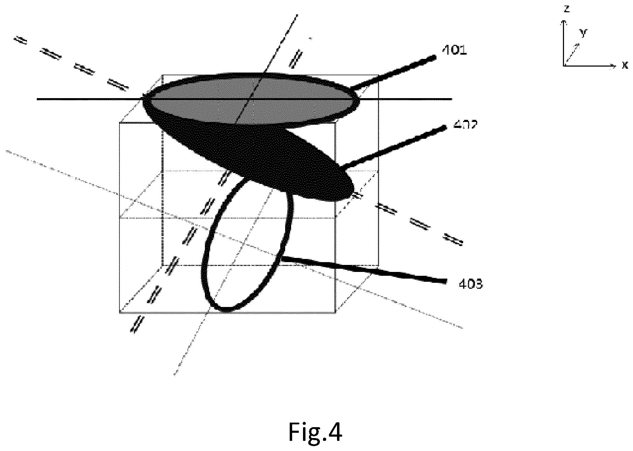

Fig 4 is a three filter arrangement comprising a planar arrangement filter **401** according to figure 2a plus a second tilted filter **402** representing the subunit **202** in figure 2b plus a third tilted filter **403** representing filter subunit **203** in figure 2c.

The angle of tilt of the interference filter with respect to the xy surface, i.e. the angle of alpha α in Figure 2c respectively beta β in fig 2b has a variable values. When measuring a filter setup with α=β=30 degree as compared to a plane filter in xy-surface orientation one observes at least 2-3 fold reduction in stray light. Furthermore varying the angle of α=β=30 to is about twofold more effective in reducing the stray light when compared to a tilted filter combination with α=β=15 degrees tilt.

The device of the present disclosure is an improvement compared to a single interference filter design. In particular, it comprises more than one interference filter. At least two interference filters are used in tandem and tilted at different angles with respect to the emitted light. in such a way that their angle β is different from degree and they a tilted by about 15 to 30 degree in the axis of the tilting angle with respect to the direction of the emitted light. Furthermore when the two filters are arranged in such a way the tilting axis of filter **1** is rotated by 90 degrees with respect to the tilting angle of the filter **1.** It is surprisingly observed that a combination of two filters and leads to a much improved and more effective filtering of the stray light coming from the device. Even more effective in filtering out stray light is shown in figure 4 in a combination of a planar arranges filter **401** combined with a first tilted filter **402** and combine with a second tilted and by 90 degree tilted filter **403.**

**Table 1**

| | Planar filter fig as in 2a | Triple filter as in fig 4 |
|---|---|---|
| Measurement device background | 2.4117 | 0.0130 |
| Sample Signal net | 7.2383 | 0.1026 |
| Standard deviation background | 0.1723 | 0.0008 |
| Signal-to-noise ( = Sample signal divided by 3 times | 14.0 | 45.3 |
| standard deviation background) | | |

The summary examples summarized in table 1 show a much reduced background that originates form residual excitation light derived entering the detector light. When comparing the effectiveness of blocking the triple filter unit is about 70 times more effective blocking unwanted straylight. Furthermore the variation of the background signal reduces in a similar way an allows as such to detect even very low signals from background. The same applies to the background signal which is reduced and an improvement of the Signal to noise ratio form 14.0 for the one filter device to 45.3 for the three filter device, i.e. by at least a factor of 3.

## Claims

1. Biosensor device adapted to detect fluorescing bounded analytes, said device comprises a light emitting means adapted to irradiate the fluorescing bounded analytes at an emitted wavelength, and a detector (103) adapted to detect a fluorescence of said bound analytes at the emitted wavelength, wherein said bound analyte is bound to a fluorescently labelled ligand so as to form a fluorescent complex.
**Characterized in that** it comprises :
- a planar interference filter (201, 401) arranged at an angle orthogonal to the optical pathway or at an angle with regards to the light pathway larger than 10° and different from 90°, adapted to specifically absorb or attenuate the emitted light,
- a first tilted interference filter (202, 402) and a second tilted interference filter (203, 403), said first and second titled interference filters being oriented so that the plane of the corresponding surfaces is titled with regards to the optical pathway at an angle larger than 10° and different from 90°, said first and second titled interference filters having both an orientation different from the one of said planar interference filter and adapted to absorb the emitted light at the wavelength of said emitted light, and wherein the tilted axis of said first (202, 402) and second (203, 403) tilted interference filters are rotated from each by more than 15 degrees and less than 180 degrees, and allowing to filter out a majority of the wavelengths of the emitted light so as to enrich it with wavelengths corresponding to the bound analytes.

2. Device according to claim 1 wherein said first (202, 402) and second (203, 403) tilted interference filters, or wherein said planar interference filter (201), first (202, 402) and second tilted interference filters are combined so as to form one interference unit.

3. Device according to one of the previous claims wherein said first and second tilted interference filters are tilted by an angle larger than 20 degrees or larger than 30 degrees with respect to the optical axis.

4. Device according to one of the previous claims and the tilting angles are rotated against each other by more than 30 and less than 120 degrees.

5. Device according to one of the previous claims and the tilting angles are rotated against each other by 90 degrees.

6. Device according to one of claims 1 to 5, wherein the angles β and α by which said tilted filters are tilted against the surface orthogonal to the optical axis are selected such that α = β > 10 degree and α = β < 80 degree, or such that α = β is between 20 and 60 degrees or between 30 and 45 degrees.

7. A method of determining the presence of a tested molecule, said method comprising the steps of
- complexing the tested molecule with a fluorescent ligand and immobilizing the resulting complex of tested molecule and fluorescent ligand on a surface,
- irradiating the immobilized complex of tested molecule and fluorescent ligand with an emitted light at a predetermined wavelength, and
- detecting the resulting fluorescence of the complex of tested molecule and its bound fluorescent ligand,
**Characterized in that** the irradiation and detection steps are performed by means of the device of one of claims 1 to 6 wherein the emitted light is attenuated at the wavelength of the light emission by means of at least one optical filter.

8. method according to claim 7, **characterized in that** the wavelength of the emitted light differs from the wavelength of the light emitted by the complex of tested molecule and fluorescent ligand under the emitted light.

9. Method according to one of claims 7 or 8, **characterized in that** the steps of irradiating the immobilized complex of tested molecule and fluorescent ligand and detecting the resulting fluorescence of the complex of tested molecule and its bound fluorescent ligand, occurs in the presence of unbounded ligands.

10. method according to one of claims 7 to 9, **characterized in that** said method is free of washing steps.

11. method according to one of claims 7 to 10, **characterized in that** it is used as diagnostic method.

## Patentansprüche

1. Biosensorvorrichtung, ausgebildet zum Nachweis fluoreszierender gebundener Analyten, wobei die Vorrichtung ein lichtemittierendes Mittel aufweist, das zum Bestrahlen der fluoreszierenden gebundenen Analyten bei einer emittierten Wellenlänge eingerichtet ist, und einen Detektor (103) umfasst, der zum Nachweis einer Fluoreszenz der gebundenen Analyten bei der emittierten Wellenlänge ausgebildet ist, wobei der gebundene Analyt an einen fluoreszierend markierten Liganden gebunden ist, um einen fluoreszierenden Komplex zu bilden.
**Gekennzeichnet dadurch, dass** die Vorrichtung umfasst:
- einen planaren Interferenzfilter (201, 401), der in einem Winkel orthogonal zum optischen Pfad oder in einem Winkel in Bezug auf den optischen Pfad angeordnet ist, der größer als 10° beträgt und sich von 90° unterscheidet, und der eingerichtet ist, dass emittierte Licht spezifisch zu absorbieren oder abzuschwächen,
- ein erstes abgewinkeltes Interferenzfilter (202, 402) und ein zweites abgewinkeltes Interferenzfilter (203, 403), wobei das erste und das zweite abgewinkelte Interferenzfilter so ausgerichtet sind, dass die Ebene der entsprechenden Oberflächen in Bezug auf den optischen Pfad in einem Winkel größer als 10° und verschieden von 90° abgewinkelt ist, wobei das erste und das zweite abgewinkelte Interferenzfilter sowohl eine Ausrichtung haben, die sich von der des planaren Interferenzfilters unterscheidet, als auch ausgebildet sind, um das emittierte Licht bei der Wellenlänge des emittierten Lichts zu absorbieren, und wobei die geneigte Achse des ersten (202, 402) und des zweiten (203, 403) abgewinkelte Interferenzfilters um mehr als 15° und weniger als 180° zueinander gedreht sind und es ermöglichen, einen Großteil der Wellenlängen des emittierten Lichts herauszufiltern, um es mit Wellenlängen anzureichern, die den gebundenen Analyten entsprechen.

2. Vorrichtung nach Anspruch 1, wobei das erste (202, 402) und das zweite (203, 403) abgewinkelte Interferenzfilter, oder wobei das planare Interferenzfilter (201), das erste (202, 402) und das zweite geneigte Interferenzfilter derart kombiniert sind, dass sie eine Interferenzeinheit bilden.

3. Vorrichtung nach einem der vorhergehenden Ansprüche, wobei das erste und das zweite abgewinkelte Interferenzfilter um einen Winkel von mehr als 20° oder mehr als 30° in Bezug auf die optische Achse geneigt sind.

4. Vorrichtung nach einem der vorhergehenden Ansprüche, wobei die Kippwinkel um mehr als 30° und weniger als 120° gegeneinander verdreht sind.

5. Vorrichtung nach einem der vorhergehenden Ansprüche, wobei die Kippwinkel um 90° gegeneinander verdreht sind.

6. Vorrichtung nach einem der Ansprüche 1 bis 5, wobei die Winkel β und α, um die die abgewinkelten Filter gegen die zur optischen Achse orthogonale Fläche abgewinkelt werden, so gewählt werden, dass α = β > 10° und α = β < 80° ist, oder so, dass α = β zwischen 20° und 60° oder zwischen 30° und 45° liegt.

7. Verfahren zur Bestimmung der Anwesenheit eines geprüften Moleküls, wobei das Verfahren die folgenden Schritte umfasst:
- Komplexieren des geprüften Moleküls mit einem fluoreszierenden Liganden und Immobilisierung des resultierenden Komplexes aus geprüftem Molekül und fluoreszierendem Liganden auf einer Oberfläche,
- Bestrahlen des immobilisierten Komplexes aus dem geprüften Molekül und dem fluoreszierenden Liganden mit einem emittierten Licht einer vorbestimmten Wellenlänge, und
- Detektieren der resultierenden Fluoreszenz des Komplexes aus dem geprüften Molekül und seinem gebundenen fluoreszierenden Liganden,
**dadurch gekennzeichnet, dass** die Bestrahlungs- und Detektionsschritte mittels der Vorrichtung nach einem der Ansprüche 1 bis 6 durchgeführt werden, wobei das emittierte Licht bei der Wellenlänge der Lichtemission mittels mindestens eines optischen Filters abgeschwächt wird.

8. Verfahren nach Anspruch 7, **gekennzeichnet durch** die Wellenlänge des emittierten Lichts sich von der Wellenlänge des Lichts unterscheidet, das von dem Komplex aus dem geprüften Molekül und dem fluoreszierenden Liganden unter dem emittierten Licht emittiert wird.

9. Verfahren nach einem der Ansprüche 7 oder 8, **gekennzeichnet durch** die Schritte des Bestrahlens des immobilisierten Komplexes aus dem geprüften Molekül und dem fluoreszierenden Liganden und des Nachweises der resultierenden Fluoreszenz des Komplexes aus dem geprüften Molekül und seinem gebundenen fluoreszierenden Liganden in Anwesenheit von nicht gebundenen Liganden erfolgen.

10. Verfahren nach einem der Ansprüche 7 bis 9, **dadurch gekennzeichnet, dass** das Verfahren frei von Waschschritten ist.

11. Verfahren nach einem der Ansprüche 7 bis 10, **dadurch gekennzeichnet, dass** es als diagnostisches Verfahren eingesetzt wird.

## Revendications

1. Dispositif biosenseur adapté pour détecter des analytes fluorescents liés, ledit dispositif comprenant des moyens d'émission de lumière adaptés pour irradier les analytes fluorescents liés à une longueur d'onde émise, et un détecteur (103) adapté pour détecter une fluorescence desdits analytes liés à la longueur d'onde émise, où ledit analyte est lié à un ligand marqué de façon fluorescente de sorte à former un complexe fluorescent,
**caractérisé en ce qu'**il comprend :
- un filtre d'interférence planaire (201, 401) disposé à un angle orthogonal au parcours optique ou à un angle supérieur à 10° et différent de 90° par rapport au parcours optique, adapté pour absorber spécifiquement ou atténuer la lumière émise,
- un premier filtre d'interférence incliné (202,402) et un second filtre d'interférence incliné (203, 403), lesdits premier et second filtres d'interférence inclinés étant orientés de sorte que le plan des surfaces correspondantes est incliné par rapport au parcours optique d'un angle supérieur à 10° et différent de 90°, lesdits premier et second filtres d'interférence inclinés ayant tous deux une orientation différente de celle du filtre d'interférence planaire et adaptés pour absorber la lumière émise à la longueur d'onde de ladite lumière émise, et où les axes inclinés desdits premier (202, 402) et second (203, 403) filtres d'interférence inclinés sont tournés l'un par rapport à l'autre de plus de 15 degrés et de moins de 180 degrés, et permettant de filtrer une majorité des longueurs d'ondes de la lumière émise de sorte à l'enrichir avec les longueurs d'onde correspondant aux analytes liés.

2. Dispositif selon la revendication 1, où le premier (202, 402) et le second (203, 403) filtres d'interférence inclinés, ou où lesdits filtres d'interférence planaire (201), premier (202, 402) et second filtres d'interférence inclinés sont combinés de sorte à former une unité d'interférence.

3. Dispositif selon l'une des revendications précédentes, où lesdits premier er second filtres d'interférence inclinés sont inclinés d'un angle supérieur à 20 degrés ou supérieur à 30 degrés par rapport à l'axe optique.

4. Dispositif selon l'une des revendications précédentes et les angles d'inclinaison sont tournés l'un par rapport à l'autre de plus de 30 et de moins de 120 degrés.

5. Dispositif selon l'une des revendications précédentes et les angles d'inclinaison sont tournés l'un par rapport à l'autre de 90 degrés.

6. Dispositif selon l'une des revendications 1 à 5, où les angles β et α selon lesquels lesdits filtres inclinés sont inclinés par rapport à la surface orthogonale à l'axe optique sont sélectionnés de sorte que α = β > 10 degrés et α = β < 80 degrés, ou de sorte que α = β est entre 20 et 60 degrés ou entre 30 et 45 degrés.

7. Une méthode de détermination de la présence d'une molécule testée, ladite méthode comprenant les étapes de
- complexer la molécule testée avec un ligand fluorescent et immobiliser le complexe résultant de la molécule testée et du ligand fluorescent sur une surface,
- irradier le complexe immobilisé de la molécule testés et du ligand fluorescent avec une lumière émise à une longueur d'onde prédéterminée, et
- détecter la fluorescence résultante du complexe de la molécule testée et sont ligand fluorescent lié,
**caractérisée en ce que** les étapes d'irradiation et de détection sont effectuées au moyen du dispositif de l'une des revendications 1 à 6 où la lumière émise est atténuée à la longueur d'onde de l'émission de lumière au moyen d'au moins un filtre optique.

8. Méthode selon la revendication 7, **caractérisée en ce que** la longueur d'onde de la lumière émise diffère de la longueur d'onde émise par le complexe de la molécule testée et du ligand fluorescent sous la lumière émise.

9. Méthode selon l'une des revendications 7 et 8, **caractérisée en ce que** les étapes d'irradiation du complexe immobilisé de la molécule testée et du ligand fluorescent et de détection de la fluorescence résultante de la molécule testée et de son ligand fluorescent lié, intervient en présence de ligands non liés.

10. Méthode selon l'une des revendications 7 à 9, **caractérisée en ce que** ladite méthode est exempte d'étapes de lavage.

11. Méthode selon l'une des revendications 7 à 10, **caractérisée en ce qu'**elle est utilisée comme une méthode de diagnostic.
